# EUROPEAN PATENT APPLICATION

(11) **EP 3 415 498 A1**
(43) Date of publication of application: **19.12.2018**
(21) Application number: 17175439.3
(22) Date of filing: 12.06.2017
(51) Int. Cl.: C07C 309/73, C07C 311/49, C07C 317/28, C07D 257/04, C07C 251/48, C07F 9/09

(54) **A DEVELOPING AGENT PRECURSOR FOR LASER MARKABLE COMPOSITIONS**

(71) Applicant: AGFA NV, 2640 Mortsel (BE); Agfa-Gevaert, 2640 Mortsel (BE)
(72) Inventor: Loccufier, Johan, 2640 Mortsel (BE)
(74) Representative: Viaene, Kris

(57) **Abstract**

The invention related to novel developing agent precursors and to laser markable compositions comprising such developing agent precursors.

## Description

### Technical field of the Invention

The present invention relates to laser markable compositions and to developing agent precursors for such laser markable compositions.

### Background art for the invention

Laser marking, i.e. providing information on for example packaging or a security document by means of a laser, is gaining interest as an answer to an increasing demand for personalization, mass customization, security, traceability and anti-counterfeiting.

Several laser marking technologies co-exist in the field.

Laser induced carbonization is one of the main technologies. However, laser marking based on carbonization is limited to black and white images.

The use of metal oxides, for example ammonium octamolybdate or molybdenum trioxide, has been developed as an alternative approach for laser marking, as disclosed in for example WO2002/074548 (Datalase) or WO2008/075101 (Siltech). However, also this laser marking technology is limited to black and white images.

WO2013/014436 (Datalase) discloses a diacetylene-based technology enabling multicolour laser marking.

Another multicolour laser marking technology makes use of leuco dyes, as disclosed in for example EP-A 2648920 (Agfa Gevaert).

Leuco dye based technologies require the use of a developing agent. A developing agent is capable of reacting with a colourless leuco dye resulting in the formation of a coloured dye.

The developing agent may not react, or at least not substantially, with the leuco dye before laser marking to avoid background coloration. The developing agent and the leuco dye thus have to be shielded from each other before laser marking.

One way to achieve the shielding is the encapsulation of the leuco dye and/or the developing agent. Upon heat treatment or exposure to IR radiation, the capsules rupture, whereupon the leuco dye and the developing agent may react with each other thereby forming a colour. Encapsulation of the leuco dye and/or the developing agent is typically used in aqueous laser markable compositions, as disclosed in WO2016/184881 (Agfa Gevaert).

Another way to achieve such a shielding is by using a so-called developing agent precursor, which does not react with the leuco dye. Upon heat treatment or infrared laser exposure, the developing agent precursor releases a developing agent, which may react with the leuco dye thereby forming a colour.

Such developing agent precursors are typically used in solvent and UV based laser markable compositions, as the encapsulation approach mentioned above is more difficult to implement in non-aqueous compositions.

Developing agent precursors are typically acid precursors. Acid precursors are chemically converted into an acid upon heat treatment or infrared laser exposure. The acid then may react with a leuco dye.

Several acid precursors have been disclosed in the patent literature.

Acid precursors are often derived from strong acids as disclosed in WO2015/091688 (Agfa Gevaert) and WO2007/088104 (Ciba Specialty Chemicals Holding), resulting in precursors with a limited thermal and hydrolytical stability on one hand and a toxicological problem induced by their reactivity on the other hand.

Other classes of thermal acid generators are based on the thermal degradation of tertiary alkyl carbonates, as disclosed in EP-A 2722367 (Agfa Gevaert) and EP-A 0605149 (Nippon Paper Industries). This type of acid precursor often has a limited reactivity upon laser exposure and generates a lot of gas upon thermal degradation, resulting in layer damage and ablation at the high laser powers necessary to obtain enough reactivity.

Therefore, there still is a need for acid precursors with a broad scope of applicability and a high reactivity without the formation of large amounts of gas.

### Summary of the invention

It an object of the invention to provide novel developing agent precursors, wherewith laser marking compositions having good laser marking properties may be prepared.

This object is realised with the developing agent precursor as defined in claim 1.

Further objects of the invention will become apparent from the description hereinafter.

### Detailed description of the invention

### Definitions

The term "monofunctional" in e.g. monofunctional polymerizable compound means that the polymerizable compound includes one polymerizable group.

The term "difunctional" in e.g. difunctional polymerizable compound means that the polymerizable compound includes two polymerizable groups.

The term "polyfunctional" in e.g. polyfunctional polymerizable compound means that the polymerizable compound includes more than two polymerizable groups.

The term "alkyl" means all variants possible for each number of carbon atoms in the alkyl group i.e. methyl, ethyl, for three carbon atoms: n-propyl and isopropyl; for four carbon atoms: n-butyl, isobutyl and tertiary-butyl; for five carbon atoms: n-pentyl, 1,1-dimethyl-propyl, 2,2Dimethylpropyl and 2-methyl-butyl, etc.

Unless otherwise specified a substituted or unsubstituted alkyl group is preferably a C₁ to C₆-alkyl group.

Unless otherwise specified a substituted or unsubstituted alkenyl group is preferably a C₂ to C₆-alkenyl group.

Unless otherwise specified a substituted or unsubstituted alkynyl group is preferably a C₂ to C₆-alkynyl group.

Unless otherwise specified a substituted or unsubstituted aralkyl group is preferably a phenyl or naphthyl group including one, two, three or more C₁ to C₆-alkyl groups.

Unless otherwise specified a substituted or unsubstituted alkaryl group is preferably a C₇ to C₂₀-alkyl group including a phenyl group or naphthyl group.

Unless otherwise specified a substituted or unsubstituted aryl group is preferably a phenyl group or naphthyl group

Unless otherwise specified a substituted or unsubstituted heteroaryl group is preferably a five- or six-membered ring substituted by one, two or three oxygen atoms, nitrogen atoms, sulphur atoms, selenium atoms or combinations thereof.

The term "substituted", in e.g. substituted alkyl group means that the alkyl group may be substituted by other atoms than the atoms normally present in such a group, i.e. carbon and hydrogen. For example, a substituted alkyl group may include a halogen atom or a thiol group. An unsubstituted alkyl group contains only carbon and hydrogen atoms

Unless otherwise specified a substituted alkyl group, a substituted alkenyl group, a substituted alkynyl group, a substituted aralkyl group, a substituted alkaryl group, a substituted aryl and a substituted heteroaryl group are preferably substituted by one or more constituents selected from the group consisting of methyl, ethyl, n-propyl, isopropyl, n-butyl, isobutyl and tertiary-butyl, ester, amide, ether, thioether, ketone, aldehyde, sulfoxide, sulfone, sulfonate ester, sulphonamide, -Cl, -Br, -I, -OH, -SH, -CN and -NO₂.

### Developing agent precursor

The developing agent precursor according to the present invention has a chemical structure according to Formula I, 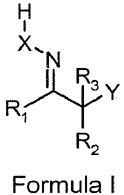 wherein,
R₁, R₂ and R₃ are selected from the group consisting of a hydrogen, a substituted or unsubstituted alkyl group, a substituted or unsubstituted alkenyl group, a substituted or unsubstituted alkynyl group, a substituted or unsubstituted alkaryl group, a substituted or unsubstituted aralkyl group, a substituted or unsubstituted aryl group and a substituted or unsubstituted heteroaryl group,
X is selected from the group consisting of an oxygen atom, a sulfur atom, -N(C=Z)R₄ and -NSO₂R₅,
Y represents a group capable of generating an acid HY having a pKₐ of 5 or less,
Z represents O or S,
   R₄ is selected from the group consisting of a hydrogen, a substituted or unsubstituted alkyl group, a substituted or unsubstituted alkenyl group, a substituted or unsubstituted alkynyl group, a substituted or unsubstituted alkaryl group, a substituted or unsubstituted aralkyl group, a substituted or unsubstituted aryl group, a substituted or unsubstituted heteroaryl group, -OR₆ and -NR₇R₈,
   R₅ and R₆ are independently selected from the group consisting of a substituted or unsubstituted alkyl group, a substituted or unsubstituted alkenyl group, a substituted or unsubstituted alkynyl group, a substituted or unsubstituted alkaryl group, a substituted or unsubstituted aralkyl group, a substituted or unsubstituted aryl group and a substituted or unsubstituted heteroaryl group,
   R₇ and R₈ are independently selected from the group consisting of a hydrogen, a substituted or unsubstituted alkyl group, a substituted or unsubstituted alkenyl group, a substituted or unsubstituted alkynyl group, a substituted or unsubstituted alkaryl group, a substituted or unsubstituted aralkyl group, a substituted or unsubstituted aryl group and a substituted or unsubstituted heteroaryl group,
   R₁ and R₂, R₁ and R₃, R₂ and R₃, and R₇ and R₈ together may represent the necessary atoms to form a 5 to 8 membered ring.

In a preferred embodiment, X is selected from the group consisting of O and NSO₂R₅.

In all embodiments described above, Y preferably represents a group capable of generating an acid HY having a pKa of 4 or less, more preferably a pKa of 3 or less.

In all these embodiments, Y is more preferably selected from the group consisting of O(CO)R₉, OSO₂R₁₀, O(PO)OR₁₁OR_{12,} SR₁₃ and SO₂R₁₄ wherein
Rg is selected from the group consisting of a hydrogen, a substituted or unsubstituted alkyl group, a substituted or unsubstituted alkenyl group, a substituted or unsubstituted alkynyl group, a substituted or unsubstituted alkaryl group, a substituted or unsubstituted aralkyl group, a substituted or unsubstituted aryl group, a substituted or unsubstituted heteroaryl group and COR₁₅,
R₁₀, R₁₁, R₁₂ and R₁₄ are independently selected from the group consisting of a substituted or unsubstituted alkyl group, a substituted or unsubstituted alkenyl group, a substituted or unsubstituted alkynyl group, a substituted or unsubstituted alkaryl group, a substituted or unsubstituted aralkyl group, a substituted or unsubstituted aryl group and a substituted or unsubstituted heteroaryl group, R₁₃ is selected from the group consisting of a substituted or unsubstituted aryl group and a substituted or unsubstituted heteroaryl group,
R₁₅ is selected from the group consisting of a hydrogen, a substituted or unsubstituted alkyl group, a substituted or unsubstituted alkenyl group, a substituted or unsubstituted alkynyl group, a substituted or unsubstituted alkaryl group, a substituted or unsubstituted aralkyl group, a substituted or unsubstituted aryl group, a substituted or unsubstituted heteroaryl group and OR₁₆,
R₁₆ is selected from the group consisting of a substituted or unsubstituted alkyl group, a substituted or unsubstituted alkenyl group, a substituted or unsubstituted alkynyl group, a substituted or unsubstituted alkaryl group, a substituted or unsubstituted aralkyl group, a substituted or unsubstituted aryl group and a substituted or unsubstituted heteroaryl group,
R₁₁ and R₁₂ may represent the necessary atoms to form a 5 to 8 membered ring.

In a most preferred embodiment, Y represents a -O(CO)COR₁₅ group or a SO₂R₁₄ group.

In another preferred embodiment, R₁ represents a substituted or unsubstituted aryl group.

In a further preferred embodiment, R₂ and R₃ are independently selected from the group consisting of a hydrogen, a substituted or unsubstituted alkyl group and a substituted or unsubstituted aryl group.

Examples of the developing agent precursor are given in Table 1 below, without being limited thereto.

**Table 1**

| | |
|---|---|
| 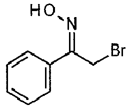 | AP-1 |
| 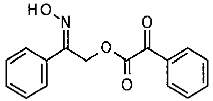 | AP-2 |
| 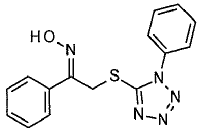 | AP-3 |
| 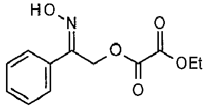 | AP-4 |
| 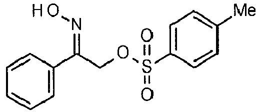 | AP-5 |
| 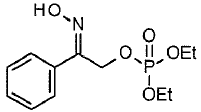 | AP-6 |
| 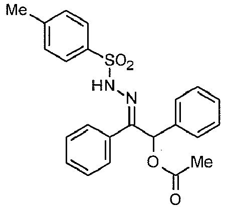 | AP-7 |
| 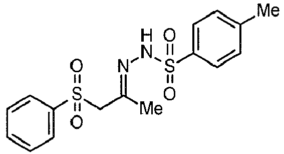 | AP-8 |
| 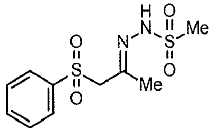 | AP-9 |
| 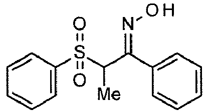 | AP-10 |
| 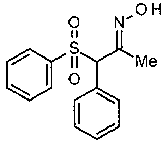 | AP-11 |
| 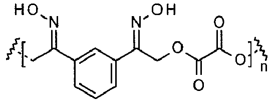 | AP-12 |
| 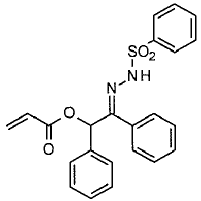 | AP-13 |
| 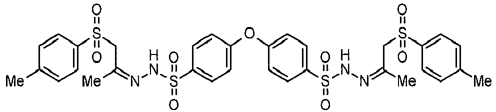 | AP-14 |
| 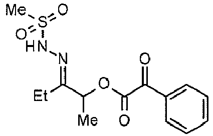 | AP-15 |
| 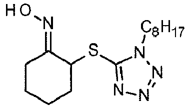 | AP-16 |
| 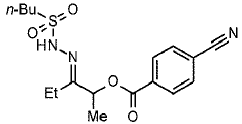 | AP-17 |
| 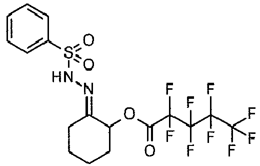 | AP-18 |

### Laser markable composition

The laser markable composition according to the present invention comprises a leuco dye and a developing agent precursor as disclosed above.

The laser markable composition preferably comprises an optothermal converting agent.

The composition may further comprise other ingredients such as an acid scavenger and a UV absorber.

The laser markable composition may also comprise a dye or pigment that enhances the contrast between the laser marked image and the background colour.

### Leuco dye

A leuco dye is a substantially colourless compound, which may form a coloured dye upon inter- or intramolecular reaction. The inter- or intramolecular reaction may be triggered by heat formed during exposure with an infrared (IR) laser.

Examples of leuco dyes that may be used are disclosed in WO2015/165854 (Agfa Gevaert), paragraphs [069] to [093].

### Optothermal converting agent

An optothermal converting agent generates heat upon absorption of radiation.

The optothermal converting agent preferably generates heat upon absorption of infrared (IR) radiation, more preferably near infrared (NIR) radiation. NIR radiation has a wavelength between 750 and 2500 nm.

Optothermal converting agents may be an infrared absorbing dye, an infrared absorbing pigment, or a combination thereof.

The optothermal converting agent is preferably an infrared absorbing dye, more preferably a NIR absorbing dye.

### Infrared radiation absorbing pigment

Suitable examples of infrared absorbing (IR) pigments include but are not limited to carbon black such as acetylene black, channel black, furnace black, lamp black, and thermal black; oxides, hydroxides, sulfides, sulfates and phosphates of metals such as copper, bismuth, iron, nickel, tin, zinc, manganese, zirconium, tungsten, lanthanum, and antimony including lanthane hexaboride, indium tin oxide (ITO) and antimony tin oxide, titanium black and black iron oxide.

A preferred infrared absorbing pigment is carbon black.

The particle size of the pigment is preferably from 0.01 to 5 µm, more preferably from 0.05 to 1µm.

The amount of the infrared absorbing pigment is between 10 and 1000 ppm, preferably between 25 and 750 ppm, more preferably between 50 and 500 ppm, most preferably between 100 and 250 ppm, all relative to the total dry weight of a laser markable layer. An amount of infrared absorbing pigment above 1000 ppm results in a too high background density of the laser markable article.

The IR dyes disclosed below may also be used as IR pigments, for example cyanine pigment, merocyanine pigment, etc.

Other suitable infrared radiation absorbing pigments are disclosed in WO2005/068207, WO2007/141522, WO2009/059900, and WO2015/015200.

### Infrared radiation absorbing dye

Infrared absorbing (IR) dyes are preferred for their narrow absorption spectra, compared to pigments, enabling multicolour images to be formed.

In principle any IR dye may be used, for example the IR dyes disclosed in "Near-Infrared Dyes for High Technology Applications" (ISBN 978-0-7923-5101-6).

An advantage of using IR dyes is that the absorption spectrum of an IR dye tends to be narrower than that of an IR pigment. This allows the production of a multicoloured image when a plurality of laser markable layers, each laser markable layer containing different IR dyes and leuco dyes, are used. The IR dyes having a different maximum absorption wavelength can then be adressed by IR lasers with corresponding emission wavelengths causing colour formation only in the laser markable layer of the adressed IR dye. Such multicolour articles have been disclosed in for example US 4720449, EP-A 2719540 and EP-A 2719541.

Preferred IR dyes are polymethine dyes due to their low absorption in the visible region and their selectivity, i.e. narrow absorption peak in the infrared region. Particular preferred polymethine IR dyes are cyanine IR dyes.

Preferred IR dyes having an absorption maximum of more than 1100 nm are those disclosed in EP-A 2722367, paragraphs [0044] to [0083] and WO2015/165854, paragraphs [0040] to [0051].

IR dyes having an absorption maximum between 1000 nm and 1100 nm are preferably selected from the group consisting of quinoline dyes, indolenine dyes, especially a benzo[cd]indoline dye. A particularly preferred IR dye is 5-[2,5-bis[2-[1-(1-methylbutyl)-benz[cd]indol-2(1H)-ylidene]ethylidene]-cyclopentylidene]-1-butyl-3-(2-methoxy-1-methylethyl)- 2,4,6(1H,3H,5H)-pyrimidinetrione (CASRN 223717-84-8) represented by the Formula IR-1, or the IR dye represented by Formula IR-2:

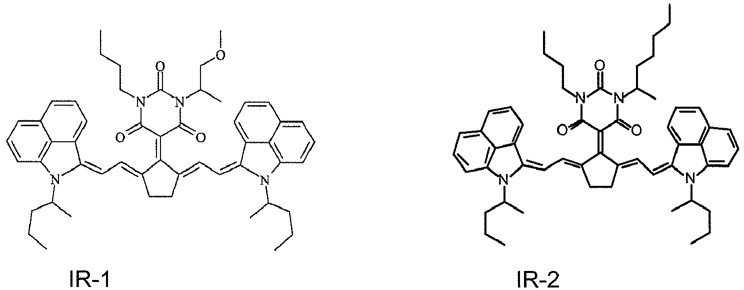

Both IR dyes IR-1 and IR-2 have an absorption maximum λₘₐₓ around 1052 nm making them very suitable for a Nd-YAG laser having an emission wavelength of 1064 nm.

A combination of two, three or more IR dyes may be used in the laser markable layer. Such a combination of IR dyes may be used to optimize the absorption spectrum of the laser markable layer. Also, a mixture of IR dyes may improve the solubility of the IR dyes in a laser markable composition, wherewith a laser markable layer is prepared.

### UV absorbers

The laser markable composition may also comprise an UV-absorber. The UV-absorber is however preferably present in a protective layer, provided on top of the printed laser markable image.

Examples of suitable UV-absorbers include 2-hydroxyphenyl-benzophenones (BP) such as Chimassorb™ 81 and Chimassorb™ 90 from BASF; 2-(2-hydroxyphenyl)-benzotriazoles (BTZ) such as Tinuvin™ 109, Tinuvin™ 1130, Tinuvin™ 171, Tinuvin™ 326, Tinuvin™ 328, Tinuvin™ 384-2, Tinuvin™ 99-2, Tinuvin™ 900, Tinuvin™ 928, Tinuvin™ Carboprotec™, Tinuvin™ 360, Tinuvin™ 1130, Tinuvin™ 327, Tinuvin™ 350, Tinuvin™ 234 from BASF, Mixxim™ BB/100 from FAIRMOUNT, Chiguard 5530 from Chitec; 2-hydroxy-phenyl-s-triazines (HPT) such as Tinuvin™ 460, Tinuvin™ 400, Tinuvin™ 405, Tinuvin™ 477, Tinuvin™ 479, Tinuvin™ 1577 ED, Tinuvin™ 1600 from BASF, 2-(2,4-dihydroxyphenyl)-4,6-bis-(2,4-dimethylphenyl)-s-triazine (CASRN1668-53-7) from Capot Chemical Ltd and 4-[4,6-bis(2-methyl-phenoxy)-1,3,5-triazin-2-yl]-1,3-benzenediol (CASRN13413-61-1); titanium dioxide such as Solasorb 100F from from Croda Chemicals; zink oxide such as Solasorb 200F from Croda Chemicals; benzoxazines such as Cyasorb UV-3638 F, CYASORB™ UV-1164 from CYTEC; and oxamides such as Sanduvor VSU from Clariant.

Preferred UV absorbers have in the wavelength region between 300 and 400 nm a maximum absorption above 330 nm, more preferably above 350 nm.

Particular preferred UV absorbers are hydroxyphenyl benzotriazoles and 2-hydroxyphenyl-s-triazines having a maximum absorption above 350 nm in the wavelength region 300 - 400 nm.

### Acid Scavenger

The laser markable composition may contain one or more acid scavengers.

Acid scavengers include organic or inorganic bases. Examples of the inorganic bases include hydroxides of alkali metals or alkaline earth metals; secondary or tertiary phosphates, borates, carbonates; quinolinates and metaborates of alkali metals or alkaline earth metals; a combination of zinc hydroxide or zinc oxide and a chelating agent (e.g., sodium picolinate); hydrotalcite such as Hycite 713 from Clariant; ammonium hydroxide; hydroxides of quaternary alkylammoniums; and hydroxides of other metals. Examples of the organic bases include aliphatic amines (e.g., trialkylamines, hydroxylamines and aliphatic polyamines); aromatic amines (e.g., N-alkyl-substituted aromatic amines, N-hydroxylalkyl-substituted aromatic amines and bis[p-(dialkylamino)phenyl]-methanes), heterocyclic amines, amidines, cyclic amidines, guanidines and cyclic guanidines.

Other preferred acid scavangers are HALS compounds. Example of suitable HALS include 292, 123, 1198, 1198 L, 144, 152, 292, 292 HP, 5100, 622 SF, 770 DF, Chimassorb™ 2020 FDL, Chimassorb™ 944 LD from BASF; Hostavin 3051, Hostavin 3050, Hostavin N 30, Hostavin N321, Hostavin N 845 PP, Hostavin PR 31 from Clariant.

Further examples of acid scavengers are salts of weak organic acids such as carboxylates (e.g. calcium stearate).

A preferred acid scavenger is an organic base, more preferably an amine. A particular preferred acid scavenger is an organic base having a pK_{b} of 7 or less.

### Laser markable Article

The laser markable article according to the present invention is prepared by applying the laser markable composition according to the present invention on a support.

The laser markable composition may be provided onto a support by co-extrusion or any conventional coating technique, such as dip coating, knife coating, extrusion coating, spin coating, spray coating, slide hopper coating and curtain coating, thereby forming a laser markable layer.

The laser markable composition may also be provided onto a support by any printing method such as intaglio printing, screen printing, flexographic printing, offset printing, inkjet printing, rotogravure printing, etc.

Using a printing method is preferred when only a part or several parts of a support has to be provided with the laser markable composition.

The laser markable article maybe selected from a packaging, a foil, a laminate, a security document, a label, a decorative object, or an RFID tag.

### Support

The laser markable composition may be applied on any type of surface, for example a metallic support, a glass support, a polymeric support, or a paper support. The laser markable composition may also be applied on a textile surface.

The support may be provided with a primer to improve the adhesion between the support and the laser markable composition.

A primer containing a dye or a pigment, for example a white primer, may also be provided on the support, for example to improve the contrast of the laser marked image.

The support may be a paper support, such as plain paper or resin coated paper, e.g. polyethylene or polypropylene coated paper.

There is no real limitation on the type of paper and it includes newsprint paper, magazine paper, office paper, or wallpaper but also paper of higher grammage, usually referred to as paper boards, such as white lined chipboard, corrugated (fiber) board and packaging board.

Also, so-called synthetic papers, such as the Synaps™ synthetic papers from Agfa Gevaert, which are opaque polyethylene terephthalate sheets, may be used as support.

Suitable polymeric supports include cellulose acetate propionate or cellulose acetate butyrate, polyesters such as polyethylene terephthalate and polyethylene naphthalate, polyamides, polycarbonates, polyimides, polyolefins, polyvinylchlorides, polyvinylacetals, polyethers, polysulfonamides, polylactide (PLA) and polyimide.

A preferred polymeric support is a biaxially stretched polyethylene terephthalate foil (PET-C foil) due to its very high durability and resistance to scratches and chemical substances.

The manufacturing of PET-C foils and supports is well-known in the art of preparing suitable supports for silver halide photographic films. For example, GB 811066 (ICI) teaches a process to produce biaxially oriented polyethylene terephthalate foils and supports.

The polymeric support may be a single component extrudate or co-extrudate. Examples of suitable co-extrudates are PET/PETG and PET/PC.

There is no restriction on the shape of the support. It can be a flat sheet, such as a paper sheet or a polymeric film or it can be a three dimensional object like e.g. a plastic coffee cup.

The three dimensional object can also be a container like a bottle or a jerry-can for including e.g. oil, shampoo, insecticides, pesticides, solvents, paint thinner or other type of liquids.

The laser markable composition may also be applied on a so-called shrink foil. Such a foil shrinks tightly over whatever it is covering when heat is applied.

The most commonly used shrink foils are polyolefin foils, i.e. polyethylene or polypropylene foils. However, other shrink foils include PCV foils.

### Packaging

A preferred laser markable article is packaging.

Laser marking is typically used to add variable data, for example batch numbers, expiry dates, addressees, etc. on the packaging.

Preferably laser marking is carried out in-line in the packaging process.

The laser marked "image" on a packaging may comprises data, images, barcodes, QR codes, or a combination thereof.

An advantage of using laser marking in a packaging process is the ability to mark information through a wrapping foil, for example the flavour-protective foil used for cigarette packs. In such a way, variable data may be provided on the cigarette packs after the protective foil has already been provided.

Another preferred laser markable packaging is used for pharmaceutical packaging. For pharmaceutical packaging, track and trace requirements become more and more demanding to comply with the ever evolving legislation.

Another advantage of using laser marking instead of another printing technique, such as inkjet printing, is the absence of any chemicals in the marking process. Especially for pharmaceutical and food packaging, the absence of chemicals in the packaging line is a great advantage.

By selecting a proper leuco dye, or a mixture of leuco dyes, the package may be provided with data or images in any colour.

A preferred packaging is folded cardboard or corrugated cardboard laminated with paper. Such packaging is preferably used for cosmetics, pharmaceuticals, food or electronics.

Multiple colour, even full colour, images may be obtained when the packaging is provided with multiple laser markable compositions, each containing a different leuco dye and optothermal converting agent, as disclosed in EP-A2719540 (Agfa Gevaert) and EP-A 2719541 (Agfa Gevaert).

### Security Documents

The laser markable compositions may also be used to prepare security documents, such as for example ID cards.

Typically, laser markable security documents are prepared by laminating a laser markable foil or laminate, optionally together with other foils or laminates, onto one or both sides of a core support.

Such laser markable security documents and their preparation have been disclosed in for example WO2015/091782 (Agfa Gevaert).

The laser markable laminate may be prepared by providing a laser markable composition according to the present invention on a support. The support is described above and is preferably a transparent polymeric support.

The laser markable laminate may comprise more than one laser markable layers or may comprise additional layers such an ink receiving layer, a UV absorbing layer, intermediate layers or adhesion promoting layers.

The laser markable laminate is typically laminated on one or both sides of a core support using elevated temperatures and pressures.

Preferred core supports are disclosed in WO2014/057018 (Agfa Gevaert), paragraphs [0112] to [0015].

The lamination temperature depends on the type of core support used. For a polyester core, lamination temperatures are preferably between 120 and 140°C, while they are preferably above 150°C -160°C for a polycarbonate core.

### Lasermarking

Laser marking is carried out with an infrared laser.

The infrared laser may be a continuous wave or a pulsed laser.

For example a CO₂ laser, a continuous wave, high power infrared laser having emission wavelength of typically 10600 nm (10.6 micrometer) may be used.

CO₂ lasers are widely available and cheap. A disadvantage however of such a CO₂ laser is the rather long emission wavelength, limiting the resolution of the laser marked information.

To produce high resolution laser marked data, it is preferred to use a near infrared (NIR) laser having an emission wavelength between 750 and 2500, preferably between 800 and 1500 nm in the laser marking step.

A particularly preferred NIR laser is an optical pumped semiconductor laser. Optically pumped semiconductor lasers have the advantage of unique wavelength flexibility, different from any other solid-state based laser. The output wavelength can be set anywhere between about 920 nm and about 1150 nm. This allows a perfect match between the laser emission wavelength and the absorption maximum of an optothermal converting agent present in the laser markable layer.

A preferred pulsed laser is a solid state Q-switched laser. Q-switching is a technique by which a laser can be made to produce a pulsed output beam. The technique allows the production of light pulses with extremely high peak power, much higher than would be produced by the same laser if it were operating in a continuous wave (constant output) mode, Q-switching leads to much lower pulse repetition rates, much higher pulse energies, and much longer pulse durations.

Laser marking may also be carried out using a so-called Spatial Light Modulator (SLM) as disclosed in WO2012/044400 (Vardex Laser Solutions).

### EXAMPLES

### Materials

All materials used in the following examples were readily available from standard sources such as ALDRICH CHEMICAL Co. (Belgium) and ACROS (Belgium) unless otherwise specified. The water used was deionized water.

**CCE** is Hydran APX-101H, a polyester urethane (45%) from DIC.

**PAR-sol** is a 40 w% aqueous solution of PAR.

**PAR** is a dimethyltrimethylolamine formaldehyde resin from Cyec Industries.

**PEA-sol** is a 10 w% dispersion of PEA in water/ethanol 1/1.

**PEA** is Topearl™ 120 from Momentive Performance Chemicals.

**Surfynsol** is a 2.5 w% solution of Surfynol™ 420.

**Surfynol**™ 420 is a non ionic surfactant from Air Products.

**DOW-sol** is a 2.5 w% solution of Dowfax™ 2A1 in isopropanol.

**Dowfax**™ **2A1** is an anionic surfactant from Pilot Chemicals C.

**Infrared Dye IR1** is an infrared dye according to the following structure.

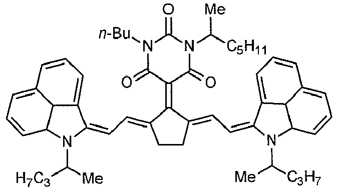

Infrared Dye IR1 can be prepared according to the synthetic methods reported in EP-A 2463109 (Agfa Gevaert), paragraphs [0150] to [0159].

**Leuco dye LD1** is a magenta leuco dye according to the following structure, supplied by Connect Chemical Production as WINCON-RED.

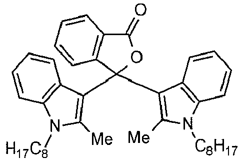

**PVB** is Pioloform BL16 supplied by Wacker-Chemie.

### Example 1

In this example, the developing agent precursors AP-1 to AP-5 and AP-7 to AP-11 are prepared.

### The synthesis of bromoacetophenone oxime (AP-1)

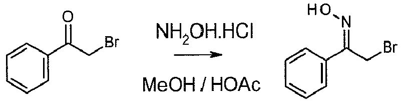

5.08 g (25 mmol) bromoacetophenone was dissolved in 50 ml methanol. 6.268 g (87.5 mmol) hydroxylamine chlorohydrate and 250 µl (4mmol) acetic acid were added. The mixture was refluxed for two hours. The mixture was allowed to cool down to room temperature and poured into 100 ml ice/water. The crude bromoacetophenone oxime precipitated from the medium. Bromoacetophenone oxime was isolated by filtration, washed with water and dried. The crude bromoacetophenone oxime was recrystallized from 100 ml hexane, isolated by filtration and dried. 2.58 g bromoacetophenone oxime was isolated (yield (y) was 48.2%, melting point (m.p.) = 88.5°C).

### The synthesis of [2-hydroxyimino-2-phenyl-ethyl] 2-oxo-2-phenyl-acetate (AP-2)

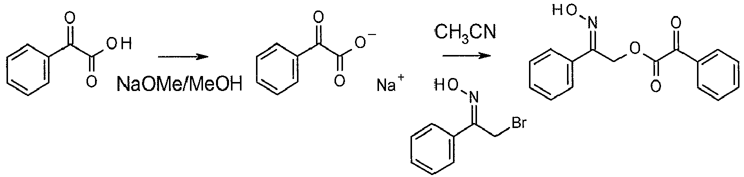

4.086 g (26.4 mmol) phenylglyoxylic acid was dissolved in 20 ml methanol. 4.757 g of a 30 wt% solution of sodium methanolate in methanol (26.4 mmol) was added dropwise. The solution was stirred for one hour at room temperature and the solvent was evaporated under reduced pressure.

1.601 g (9.3 mmol) phenylglyoxylic acid sodium salt was added to a solution of 2 g (9.3 mmol) bromoacetophenone oxime in 40 ml acetonitrile. The mixture was refluxed for one hour. The mixture was allowed to cool down to room temperature and the precipitated salts were removed by filtration. The solvent was removed under reduced pressure and [2-hydroxyimino-2-phenyl-ethyl] 2-oxo-2-phenylacetate was purified by preparative column chromatography on a GraceResolve column, using a gradient elution from methylene chloride to methylene chloride/ethyl acetate 95/5. 1.67 g of [2-hydroxyimino-2-phenyl-ethyl] 2-oxo-2-phenyl-acetate was isolated (y : 63.5 %). [2-hydroxyimino-2-phenyl-ethyl] 2-oxo-2-phenyl-acetate was analyzed by TLC chromatography on TLC Silicagel 60 F254, supplied by Merck (eluent : methylene chloride/ethyl acetate 96/4 : isomer 1 : Rf: 0.33, isomer 2 : Rf: 0.41). The structure of [2-hydroxyimino-2-phenylethyl] 2-oxo-2-phenyl-acetate was confirmed using TLC-MS.

### The synthesis of 1-phenyl-2-(1-phenyltetrazol-5-yl)sulfanyl-ethanone oxime (AP-3)

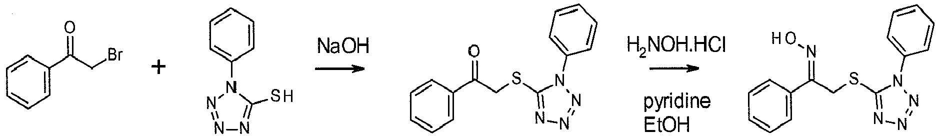

35.6 g (0.2 mol) 1-phenyl-5-mercapto-tetrazole was suspended in 500 ml water. A solution of 8 g (0.2 mol) sodium hydroxide in 150 ml water was added and the mixture was stirred for 30 minutes at room temperature. A solution of 39.8 g (0.2 mol) bromoacetophenone in 250 ml ethanol was added. The reaction was allowed to continue for 30 minutes. 1-phenyl-2-(1-phenyltetrazol-5-yl)sulfanyl-ethanone was isolated by filtration, washed with diethyl ether and dried. 52 g of 1-phenyl-2-(1-phenyltetrazol-5-yl)sulfanyl-ethanone was isolated (y : 88%, m.p. : 116°C)

2.96 g (10 mmol) 1-phenyl-2-(1-phenyltetrazol-5-yl)sulfanyl-ethanone was dissolved in 20 ml ethanol. 1.003 g (14 mmol) hydroxylamine chlorohydrate was added followed by the addition of 1.107 g (14 mmol) pyridine. The mixture was refluxed for one hour. The mixture was allowed to cool down to room temperature and the solvent was evaporated under reduced pressure. The residue was dissolved in 100 ml methylene chloride. The precipitated salts were removed by filtration and 1-phenyl-2-(1-phenyltetrazol-5-yl)sulfanyl-ethanone oxime was purified by preparative column chromatography on a GraceResolve column, using a gradient elution from methylene chloride to methylene chloride/ethyl acetate 95/5. 2.74 g of 1-phenyl-2-(1-phenyltetrazol-5-yl)sulfanyl-ethanone oxime was isolated (y : 88%). 1-phenyl-2-(1-phenyltetrazol-5-yl)sulfanyl-ethanone oxime was analyzed using TLC on TLC Silicagel 60 F254, supplied by Merck (eluent: methylene chloride/ethyl acetate 96/4 : Rf: 0.18). The structure was confirmed using TLC-MS.

### The synthesis of O1-ethyl O2-[(2E)-2-hydroxyimino-2-phenyl-ethyl] oxalate (AP-4)

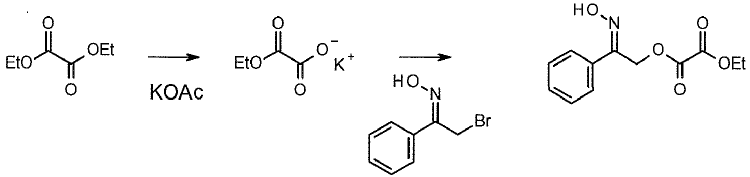

### Step 1: the synthesis of oxalic acid monoethyl ester potassium salt

14.76 g (0.1 mol) diethyl oxalate was dissolved in 20 ml ethanol. 9.82 g (0.1 mol) potassium acetate and 1.8 g (0.1 mol) water were added. The mixture was refluxed for three hours. The reaction mixture was allowed to cool down to room temperature and 20 ml methyl t.butyl ether was added. The precipitated oxalic acid monoethyl ester potassium salt was isolated by filtration and suspended in a mixture of 100 ml ethanol and 100 ml methyl t.butyl ether. Oxalic acid monoethyl ester potassium salt was isolated by filtration and dried. 12.43 g oxalic acid monoethyl ester potassium salt was isolated (y : 80 %) and used without further purification.

### Step 2 : the synthesis of 01-ethyl O2-[(2E)-2-hydroxyimino-2-phenyl-ethyl] oxalate

2 g (9.3 mmol) bromoacetophenone oxime was dissolved in 40 ml acetonitrile. 1.816 g (11.6 mmol) oxalic acid monoethyl ester potassium salt was added. The reaction was allowed to continue for 60 hours at room temperature followed by reflux for half an hour. The reaction mixture was allowed to cool down to room temperature. The precipitated salts were removed by filtration and the solvent was evaporated under reduced pressure. The crude 01-ethyl O2-[(2E)-2-hydroxyimino-2-phenyl-ethyl] oxalate was purified using preparative column chromatography on a GraceResolve column, using methylene chloride/ethyl acetate (96/4) as eluent. 1.04 g of 01-ethyl O2-[(2E)-2-hydroxyimino-2-phenylethyl] oxalate (AP-4) was isolated (y : 44%; TLC analysis on TLC Silicagel 60 F254, supplied by Merck; eluent : methylene chloride/ethyl acetate 96/4 : Rf : 0.20). The structure was confirmed using TLC-MS.

### The synthesis of [(2E)-2-hydroxyimino-2-phenyl-ethyl] 4-methylbenzenesulfonate (AP-5)

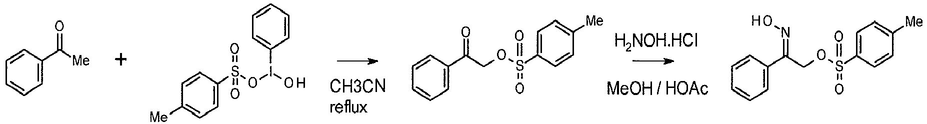

### Step 1 : the synthesis of phenacyl 4-methylbenzenesulfonate

10.11 g (25 mmol) hydroxyl(phenyl)iodo tosylate was dissolved in 65 ml acetonitrile. 3.065 g (25 mmol) acetophenone was added and the mixture was refluxed for 2 hours. The reaction mixture was allowed to cool down to room temperature and the mixture was added to 75 ml water. The mixture was extracted with 50 ml methylene chloride. The organic fraction was isolated and dried over MgSO4. The solvent was evaporated under reduced pressure and the crude phenacyl 4-methylbenzenesulfonate was purified by preparative column chromatography on a Grace Resolve column, using methylene chloride as eluent. 4.2 g of phenacyl 4-methylbenzenesulfonate was isolated (y : 58 %, TLC analysis on TLC Silicagel 60 F254, supplied by Merck; eluent: methylene chloride : Rf : 0.46). The structure was confirmed using TLC-MS.

### Step 2 : the synthesis of [(2E)-2-hydroxyimino-2-phenyl-ethyl] 4-methylbenzenesulfonate

2 g (6.9 mmol) phenacyl 4-methylbenzenesulfonate was dissolved in 20 ml methanol. 1.73 g (24.1 mmol) hydroxylamine chlorohydrate and 66 mg acetic acid were added and the mixture was refluxed for three hours. The reaction mixture was allowed to cool down to room temperature and the solvent was evaporated under reduced pressure. 50 ml methylene chloride was added to the residue and the precipitated excess of hydroxylamine chlorohydrate was removed by filtration. [(2E)-2-hydroxyimino-2-phenyl-ethyl] 4-methylbenzenesulfonate was isolated using preparative column chromatography on a GraceResolve column, using a gradient elution from methylene chloride to methylene chloride/ethyl acetate 98/2. 0.377g of [(2E)-2-hydroxyimino-2-phenylethyl] 4-methylbenzenesulfonate (AP-5) was isolated (y : 18%, TLC analysis on TLC Silicagel 60 F254, supplied by Merck; eluent: methylene chloride/ethyl acetate 94/6 : Rf: 0.55). The structure was confirmed using 1 H-NMR analysis.

### The synthesis of [(2E)-1,2-diphenyl-2-(p-tolylsulfonylhydrazono)ethyl] acetate (AP-7)

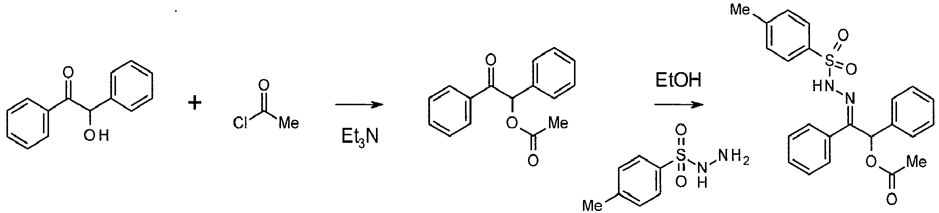

### Step 1 : the synthesis of (2-oxo-1,2-diphenyl-ethyl) acetate

4.33 g (20 mmol) benzoin was dissolved in 20 ml methylene chloride. 4.048 g (40 mmol) triethyl amine was added and the solution was cooled to 0°C. 2.403 g (30 mmol) acetyl chloride was added dropwise and the reaction was allowed to continue at room temperature for 16 hours. The reaction mixture was added to a mixture of 75 ml brine and 50 ml methylene chloride. The methylene chloride fraction was isolated, dried over MgSO4 and evaporated under reduced pressure. The crude (2-oxo-1,2-diphenyl-ethyl) acetate was purified using preparative column chromatography on a GraceResolve column, using a gradient elution from methylene chloride to methylene chloride/ethyl acetate 98/2. 3.05 g of (2-oxo-1,2-diphenyl-ethyl) acetate was isolated (y : 60 %, TLC analysis on TLC Silicagel 60 F254, supplied by Merck; eluent: methylene chloride : Rf: 0.41). The structure was confirmed using TLC-MS.

### Step 2 : the synthesis of [(2E)-1,2-diphenyl-2-(p-tolylsulfonylhydrazono)ethyl] acetate

2.543 g (10 mmol) of (2-oxo-1,2-diphenyl-ethyl) acetate was dissolved in 20 ml ethanol. 1.92 g (10 mmol) tosyl hydrazide was added. The reaction was allowed to continue at room temperature for 60 hours. The mixture was refluxed for an additional two hours. The solvent was evaporated under reduced pressure and [(2E)-1,2-diphenyl-2-(p-tolylsulfonylhydrazono)ethyl] acetate was isolated by preparative column chromatography on a GraceResolve column, using methylene chloride as eluent. 0.33 g of [(2E)-1,2-diphenyl-2-(p-tolylsulfonyl-hydrazono)ethyl] acetate was isolated (y : 8%, TLC analysis on TLC Silicagel 60 F254, supplied by Merck; eluent : methylene chloride : Rf: 0.32). The structure was confirmed using TLC-MS.

### The synthesis of N-[(E)-[2-(benzenesulfonyl)-1-methyl-ethylidene]amino]-4-methyl-benzenesulfonamide (AP-8)

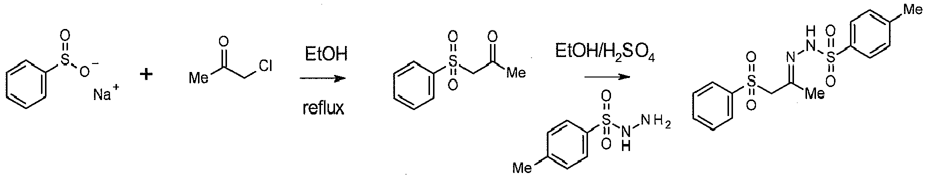

### Step 1 : The synthesis of 1-(phenylsulfonyl)-2-propanone

21.16 g (0.125 mol) benzene sulfinic acid sodium salt was dissolved in 125 ml ethanol. 12.05 g (0.125 mol) chloro-acetone was added and the reaction mixture was refluxed for six hours. The reaction mixture was allowed to cool down to room temperature. 1-(phenylsulfonyl)-2-propanone partially crystallized from the medium. The crystallized 1-(phenylsulfonyl)-2-propanone, contaminated sodium chloride, was isolated by filtration. The crude 1-(phenylsulfonyl)-2-propanone was treated with 100 ml methylene chloride. The precipitated salts were removed by filtration and the methylene chloride fraction was evaporated under reduced pressure. The residue was dissolved in a minimum methylene chloride. Hexane was added until 1-(phenylsulfonyl)-2-propanone crystallized and isolated by filtration. 5.1 g of 1-(phenylsulfonyl)-2-propanone was isolated. A second fraction was isolated from the reaction mixture by evaporating the filtrate, followed by purification of the crude 1-(phenylsulfonyl)-2-propanone by preparative column chromatography on a GraceResolve column, using a gradient elution from methylene chloride to methylene chloride/ethyl acetate 95/5. A second fraction of 7.6 g 1-(phenylsulfonyl)-2-propanone was isolated. 1-(phenylsulfonyl)-2-propanone was analyzed by TLC analysis (TLC analysis on TLC Silicagel 60 F254, supplied by Merck; eluent : methylene chloride/ethyl acetate 94/6 : Rf : 0.38).

### Step 2 : The synthesis of N-[(E)-[2-(benzenesulfonyl)-1-methyl-ethylidene]amino]-4-methyl-benzenesulfonamide

1.19 g (6 mmol) 1-(phenylsulfonyl)-2-propanone was dissolved in 50 ml ethanol. 2.458 g (13 mmol) (4-methylphenylsulfonyl)hydrazide and 1 ml concentrated sulfuric acid were added. The reaction mixture was refluxed for 1 hour. The reaction mixture was allowed to cool down to room temperature and the mixture was added to 100 ml water. The mixture was stirred for 15 minutes and the crude N-[(E)-[2-(benzenesulfonyl)-1-methyl-ethylidene]amino]-4-methyl-benzenesulfonamide was isolated by filtration, washed with water and dried. The crude N-[(E)-[2-(benzenesulfonyl)-1-methyl-ethylidene]amino]-4-methyl-benzenesulfonamide was recrystallized from ethanol. N-[(E)-[2-(benzenesulfonyl)-1-methyl-ethylidene]amino]-4-methyl-benzenesulfonamide was isolated by filtration and dried. 1.72 g (y : 78.8%) N-[(E)-[2-(benzenesulfonyl)-1-methyl-ethylidene]amino]-4-methyl-benzenesulfonamide was isolated (TLC analysis on Reveleris RP C18 TLC plates, supplied by Grace , eluent methanol/1M NaCl 60/40 : Rf: 0.38). The structure was confirmed using TLC-MS.

### The synthesis of N-[(E)-[2-(benzenesulfonyl)-1-methyl ethylidene]amino] methanesulfonamide (AP-9)

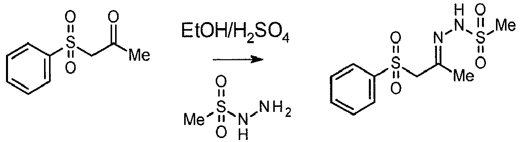

1.982 g (10 mmol) 1-(phenylsulfonyl)-2-propanone was dissolved in 85 ml ethanol. 2.423 g (22 mmol) methylsulfonylhydrazide and 1.662 ml concentrated sulfuric acid were added. The reaction mixture was refluxed for 1 hour. The reaction mixture was allowed to cool down to room temperature and the mixture was added to 140 ml water. The mixture was stirred for 15 minutes and the crude N-[(E)-[2-(benzenesulfonyl)-1-methyl ethylidene]amino] methanesulfonamide was isolated by filtration, washed with water and dried. The crude N-[(E)-[2-(benzenesulfonyl)-1-methyl ethylidene]amino] methanesulfonamide was recrystallized from ethanol. N-[(E)-[2-(benzenesulfonyl)-1-methyl ethylidene]amino] methanesulfonamide was isolated by filtration and dried. 2.1 g (y : 72.4%) -[(E)-[2-(benzenesulfonyl)-1-methyl ethylidene]amino] methanesulfonamide was isolated. The structure was confirmed using TLC-MS.

### The synthesis of 2-(benzenesulfonyl)-1-phenyl-propan-1-one oxime (AP-10)

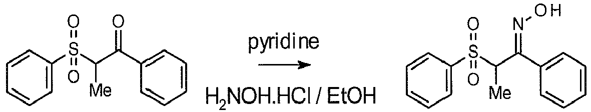

2-(benzenesulfonyl)-1-phenyl-propan-1-one was prepared according to Ramaiah et al., Indian Journal of Organic Chemistry, Section B: Organic Chemistry Including Medicinal Chemistry, 38B(3), 297-301 (1999).

2.743 g (10 mmol) 2-(benzenesulfonyl)-1-phenyl-propan-1-one was dissolved in 40 ml methanol. 2.866 g (40 mmol) hydroxyl amine chlorohydrate and 3.164 g (40 mmol) pyridine were added. The reaction mixture was refluxed for 17 hours. The reaction mixture was allowed to cool down to room temperature. The mixture was added to 75 ml water. The crude 2-(benzenesulfonyl)-1-phenyl-propan-1-one oxime separated from the mixture as a viscous oil and was isolated. The crude 2-(benzenesulfonyl)-1-phenyl-propan-1-one oxime was dissolved in 100 ml methylene chloride. The methylene chloride solution was dried over MgSO4 and evaporated under reduced pressure. 2-(benzenesulfonyl)-1-phenyl-propan-1-one oxime was purified by preparative column chromatography on GraceResolve column, using a gradient elution from methylene chloride to methylene chloride/ethyl acetate 95/5. 1.86 g (y : 64%) of 2-(benzenesulfonyl)-1-phenyl-propan-1-one oxime was isolated (TLC analysis on TLC Silicagel 60 F254, supplied by Merck; eluent : methylene chloride/ethyl acetate : Rf : 0.29 for isomer 1 and 0.23 for isomer 2). The structure of each isomer was confirmed using TLC-MS.

### The synthesis of 1-(benzenesulfonyl)-1-phenyl-propan-2-one oxime (AP-11)

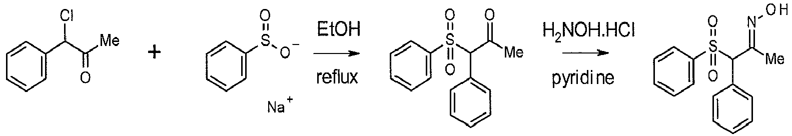

1-chloro-1-phenyl-propan-2-one was prepared according to Hemming et al., Organic Letters, 14(1), 126-129 (2012).

### Step 1 : The synthesis of 1-(benzenesulfonyl)-1-phenyl-propan-2-one

60 g (0.36 mol) benzene sulfinic acid sodium salt was dissolved in 400 ml ethanol at reflux temperature. 60 g (0.36 mol) 1-chloro-1-phenyl-propan-2-one was added in small portions over 30 minutes. The mixture was further refluxed for an additional hour. The formed sodium chloride was removed by filtration of the hot reaction mixture and 400 ml water was added to the filtrate. Upon cooling down to room temperature, 1-(benzenesulfonyl)-1-phenyl-propan-2-one crystallized from the medium. 1-(benzenesulfonyl)-1-phenyl-propan-2-one was isolated by filtration, washed with water and dried. 73 g (y : 75%) of 1-(benzenesulfonyl)-1-phenyl-propan-2-one was isolated (m.p. 122-123°C).

### Step 2 : The synthesis of 1-(benzenesulfonyl)-1-phenyl-propan-2-one oxime

2.743 g (10 mmol) 1-(benzenesulfonyl)-1-phenyl-propan-2-one was dissolved in 40 ml ethanol. 2.866 g (40 mmol) hydroxyl amine chlorohydrate and 3.164 g (40 mmol) pyridine were added. The mixture was refluxed for 16 hours. The reaction mixture was allowed to cool down to room temperature and added to 75 ml water. 1-(benzenesulfonyl)-1-phenyl-propan-2-one oxime precipitated form the medium, was isolated by filtration and dried. 1-(benzenesulfonyl)-1-phenyl-propan-2-one oxime was treated with 50 ml methylene chloride and dried, yielding 1.91 g (y : 66%) of 1-(benzenesulfonyl)-1-phenyl-propan-2-one oxime as a white crystalline powder (TLC analysis on TLC Silicagel 60 F254, supplied by Merck; eluent: methylene chloride / ethyl acetate 94/6 : Rf : 0.25). The structure was confirmed using TLC-MS.

### Example 2

This example illustrates the broad scope of structural designs according to the present invention that can be used as acid generators for laser markable compositions.

A 1100 µm thick polyethylene terephthalate sheet was first longitudinally stretched and then coated on one side with a coating composition SUB-1, as given in Table 2 at a wet coating thickness of 10 µm.

**Table 2**

| **Components (wt%)** | **SUB-1** |
|---|---|
| Deionized water | 69.44 |
| CCE | 15.40 |
| Resorcinol | 12.55 |
| PAR-sol | 0.57 |
| PEA-sol | 0.68 |
| DOW-sol | 0.68 |
| Surfynsol | 0.68 |

After drying, the longitudinally stretched and coated polyethylene terephthalate sheet was transversally stretched to produce a single side subbed 63 µm thick sheet PET-C.

The coating solutions of Table 3 were coated on the subbed side of the 63 µm thick PET-C sheet at a wet coating thickness according to Table 4 and dried for 5 minutes at 50°C in a circulation oven to obtain the laser markable laminates LML-01 to LML-10.

**Table 3**

| **Ingredients (wt%)** | **LML-01** | **LML-02** | **LML-03** | **LML-04** | **LML-05** | **LM L-06** |
|---|---|---|---|---|---|---|
| PVB | 10 | 20 | 20 | 10 | 20 | 10 |
| IR1 | 0.03 | 0.03 | 0.03 | 0.03 | 0.03 | 0.03 |
| LD1 | 13 | 9.86 | 9.25 | 9.25 | 7.0 | 2.29 |
| AP-1 | 8 | - | - | - | - | - |
| AP-2 | - | 9.28 | - | - | - | - |
| AP-3 | - | - | 9.34 | - | - | - |
| AP-4 | - | - | - | 7.54 | - | - |
| AP-5 | - | - | - | - | 6.94 | - |
| AP-7 | - | - | - | - | - | 3.14 |
| Ethyl acetate | 68.97 | 60.83 | 61.38 | 73.18 | 66.03 | 84.54 |

| **Ingedients (wt%)** | **LML-07** | **LML-08** | **LML-09** | **LML-10** | | |
|---|---|---|---|---|---|---|
| PVB | 19 | 19 | 20 | 19 | | |
| IR1 | 0.03 | 0.03 | 0.03 | 0.03 | | |
| LD1 | 3.7 | 2.95 | 9 | 3.2 | | |
| AP-8 | 4.4 | - | - | - | | |
| AP-9 | - | 2.8 | - | - | | |
| AP-10 | - | - | 8.5 | - | | |
| AP-11 | - | - | - | 3 | | |
| Ethyl acetate | - | - | 62.47 | - | | |
| MEK | 72.87 | 75.22 | - | 74.77 | | |

The laser markable laminates LML-01 to LML-10 were then laminated onto a 500 µm PETG core from WOLFEN to deliver the laser markable articles LMA-1 to LMA-10.

**Table 4**

| **LMA** | **LML** | **Coating thickness (µm)** | **% of the laser power** |
|---|---|---|---|
| LMA-1 | LML-1 | 20 | 100 |
| LMA-1 | LML-1 | 20 | 50 |
| LMA-2 | LML-2 | 20 | 50 |
| LMA-3 | LML-3 | 20 | 50 |
| LMA-4 | LML-4 | 20 | 60 |
| LMA-5 | LML-5 | 50 | 80 |
| LMA-6 | LML-6 | 50 | 70 |
| LMA-7 | LML-7 | 40 | 70 |
| LMA-8 | LML-8 | 40 | 70 |
| LMA-9 | LML-9 | 20 | 70 |
| LMA-10 | LML-10 | 40 | 70 |

The laser markable articles LMA-1 to LMA-10 were exposed with a optically pumped semiconductor laser emitting at 1064 nm (5Genesis MX 1064-10000 MTM from Coherent) to produce a magenta colored wedge of 0.5 cm x 0.5 cm square boxes of increasing optical density, using a percentage of the maximal power of the laser as given in Table 4.

The maximum density of each wedge was measured using a Barbieri Spectro 100 xy. The visual density was measured. The results are summarized in Table 5.

**Table 5**

| **Laser markable article** | **Dmax** |
|---|---|
| LMA-1 | 1.02 |
| LMA-2 | 0.53 |
| LMA-3 | 0.89 |
| LMA-4 | 0.64 |
| LMA-5 | 0.88 |
| LMA-6 | 0.47 |
| LMA-7 | 0.82 |
| LMA-8 | 0.55 |
| LMA-9 | 0.60 |
| LMA-10 | 0.58 |

From Table 5 it becomes apparent that a broad scope of acid precursors according to the present invention is useful as developing agent precursor in laser markable compositions.

## Claims

1. A developing agent precursor having a chemical structure according to Formula I 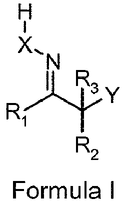 wherein,
R₁, R₂ and R₃ are selected from the group consisting of a hydrogen, a substituted or unsubstituted alkyl group, a substituted or unsubstituted alkenyl group, a substituted or unsubstituted alkynyl group, a substituted or unsubstituted alkaryl group, a substituted or unsubstituted aralkyl group, a substituted or unsubstituted aryl group and a substituted or unsubstituted heteroaryl group,
X is selected from the group consisting of an oxygen atom, a sulfur atom, -N(C=Z)R₄ and -NSO₂R₅,
Y represents a group capable of generating an acid HY having a pKₐ of 5 or less, Z represents an oxygen or a sulfur atom,
R₄ is selected from the group consisting of a hydrogen, a substituted or unsubstituted alkyl group, a substituted or unsubstituted alkenyl group, a substituted or unsubstituted alkynyl group, a substituted or unsubstituted alkaryl group, a substituted or unsubstituted aralkyl group, a substituted or unsubstituted aryl group, a substituted or unsubstituted heteroaryl group, -OR₆ and -NR₇R₈, R₅ and R₆ are independently selected from the group consisting of a substituted or unsubstituted alkyl group, a substituted or unsubstituted alkenyl group, a substituted or unsubstituted alkynyl group, a substituted or unsubstituted alkaryl group, a substituted or unsubstituted aralkyl group, a substituted or unsubstituted aryl group and a substituted or unsubstituted heteroaryl group,
R₇ and R₈ are independently selected from the group consisting of a hydrogen, a substituted or unsubstituted alkyl group, a substituted or unsubstituted alkenyl group, a substituted or unsubstituted alkynyl group, a substituted or unsubstituted alkaryl group, a substituted or unsubstituted aralkyl group, a substituted or unsubstituted aryl group and a substituted or unsubstituted heteroaryl group,
R₁ and R₂, R₁ and R₃, R₂ and R₃, and R₇ and R₈ together may represent the necessary atoms to form a 5 to 8 membered ring.

2. The developing agent precursor according to claim 1, wherein X is selected from the group consisting of O and NSO₂R₅.

3. The developing agent precursor according to claim 1 or 2, wherein Y represents a group capable of generating an acid HY having a pKa of 3 or less.

4. The developing agent precursor according to any of the preceding claims, wherein Y is selected from the group consisting of O(CO)R₉, OSO₂R₁₀, O(PO)OR₁₁OR₁₂, SR₁₃ and SO₂R₁₄ and wherein
R₉ is selected from the group consisting of a hydrogen, a substituted or unsubstituted alkyl group, a substituted or unsubstituted alkenyl group, a substituted or unsubstituted alkynyl group, a substituted or unsubstituted alkaryl group, a substituted or unsubstituted aralkyl group, a substituted or unsubstituted aryl group, a substituted or unsubstituted heteroaryl group and COR₁₅,
R₁₀, R₁₁, R₁₂ and R₁₄ are independently selected from the group consisting of a substituted or unsubstituted alkyl group, a substituted or unsubstituted alkenyl group, a substituted or unsubstituted alkynyl group, a substituted or unsubstituted alkaryl group, a substituted or unsubstituted aralkyl group, a substituted or unsubstituted aryl group and a substituted or unsubstituted heteroaryl group,
R₁₃ is selected from the group consisting of a substituted or unsubstituted aryl group and a substituted or unsubstituted heteroaryl group,
R₁₅ is selected from the group consisting of a hydrogen, a substituted or unsubstituted alkyl group, a substituted or unsubstituted alkenyl group, a substituted or unsubstituted alkynyl group, a substituted or unsubstituted alkaryl group, a substituted or unsubstituted aralkyl group, a substituted or unsubstituted aryl group, a substituted or unsubstituted heteroaryl group and OR₁₆,
R₁₆ is selected from the group consisting of a substituted or unsubstituted alkyl group, a substituted or unsubstituted alkenyl group, a substituted or unsubstituted alkynyl group, a substituted or unsubstituted alkaryl group, a substituted or unsubstituted aralkyl group, a substituted or unsubstituted aryl group and a substituted or unsubstituted heteroaryl group,
R₁₁ and R₁₂ may represent the necessary atoms to form a 5 to 8 membered ring.

5. The developing agent precursor according to any of the preceding claims, wherein Y represents a -O(CO)COR₁₅ group or a SO₂R₁₄ group, wherein R₁₄ and R₁₅ have the same meaning as in claim 4.

6. The developing agent precursor according to any of the preceding claims, wherein R₁ represents a substituted or unsubstituted aryl group.

7. The developing agent precursor according to any of the preceding claims, wherein R₂ and R₃ are independently selected from the group consisting of a hydrogen, a substituted or unsubstituted alkyl group and a substituted or unsubstituted aryl group.

8. A laser markable composition comprising a leuco dye and a developing agent precursor as defined in any of the claims 1 to 7.

9. The laser markable composition according to claim 8 further comprising an optothermal converting agent.

10. The laser markable composition according to claim 9 wherein the optothermal converting agent is an infrared absorbing dye.

11. A laser markable article comprising a laser markable composition as defined in any of the claim 8 to 10 provided on a support.

12. The laser markable article according to claim 11 wherein the laser markable composition is provided on a support by intaglio printing, screen printing, flexographic printing, offset printing, inkjet printing, or rotogravure printing.

13. The laser markable article according to claim 11 or 12 wherein the article is selected from the group consisting of a packaging, a foil, a laminate, a security document, a label, a decorative object and an RFID tag.

14. A method of preparing a laser marked article comprising the step of exposing a laser markable article as defined in any of the claims 11 to 13 with an infrared laser thereby forming a laser marked image.

15. The method according to claim 14 wherein the laser marked image is selected from the group consisting of a barcode, a QR code, alphanumerical data, pictures, and logos.
